Europäisches Patentamt

European Patent Office

Office européen des brevets

(11) Veröffentlichungsnummer : **0 455 631 B1**

(12) **EUROPÄISCHE PATENTSCHRIFT**

(45) Veröffentlichungstag der Patentschrift :
**30.06.93 Patentblatt 93/26**

(21) Anmeldenummer : **89902505.0**

(22) Anmeldetag : **25.01.89**

(86) Internationale Anmeldenummer :
**PCT/EP89/00034**

(87) Internationale Veröffentlichungsnummer :
**WO 90/08547 09.08.90 Gazette 90/19**

(51) Int. Cl.⁵ : **A61K 31/60,** A61K 9/06, A61K 9/08

(54) **SALICYLSÄUREHALTIGES MITTEL GEGEN SCHUPPENDE HAUTERKRANKUNGEN.**

(43) Veröffentlichungstag der Anmeldung :
**13.11.91 Patentblatt 91/46**

(45) Bekanntmachung des Hinweises auf die
Patenterteilung :
**30.06.93 Patentblatt 93/26**

(84) Benannte Vertragsstaaten :
**AT BE CH DE FR GB IT LI LU NL SE**

(56) Entgegenhaltungen :
**EP-A- 0 043 738**
**EP-A- 0 063 870**
**WO-A-89/00853**
**DE-C- 762 726**
**Pharmazeutische Industrie, vol. 44, no. 6, 1982
(Aulendorf) U. Adams et al., pp. 625-629**
**Journal of Pharmaceutidal Sciences, vol. 73,
no. 8, 1984 (Washington) E.R. Cooper, pp.
1153-1156**
**Chemical Abstracts, vol. 86, no. 16, 18 April
1977 (Columbus, Ohio, US) G. Zygmunt, p. 296**

(73) Patentinhaber : **DR. AUGUST WOLFF
CHEMISCH-PHARMAZEUTISCHE FABRIK
GMBH & CO. KG
Sudbrackstrasse 56
W-4800 Bielefeld 1 (DE)**

(72) Erfinder : **MINNINGER, Konrad
Hattinger Stra e 45
W-5830 Schwelm (DE)**
Erfinder : **TANG, David
Hauptstra e 304
W-4690 Herne 2 (DE)**
Erfinder : **OBERHAGEMANN, Rainer
Hauptstra e 304
W-4690 Herne 2 (DE)**
Erfinder : **KNIE, Ulrich
Beethovenstra e 19 b
W-4902 Bad Salzuflen (DE)**

(74) Vertreter : **Wolgast, Rudolf, Dipl.-Chem. Dr. et
al
Dipl.-Phys. Jürgen Weisse Dipl.-Chem. Dr.
Rudolf Wolgast Bökenbusch 41 Postfach 11 03
86
W-5620 Velbert 11 Langenberg (DE)**

EP 0 455 631 B1

**Beschreibung**

Die Erfindung betrifft ein salicylsäurehaltiges Mittel zur lokalen Therapie von schuppenden Hauterkrankungen im Bereich der Human- und Veterinärmedizin. Zu diesen schuppenden Hauterkrankungen zählen beispielsweise Psoriasis, Tinea amiantacea und Seborrhoea sicca.

Es ist beispielsweise aus E. Mutschler, "Arzneimittelwirkungen", Wissenschaftliche Verlagsgesellschaft, Stuttgart 1975, S. 373, bekannt, daß Salicylsäure die Ablösung von Schuppen und die Erweichung von Hornmaterial bewirkt und in Form von Salicylvaseline, Salicylspiritus und Salicylkollodium zur Anwendung kommt. Als weitere Mittel zur Behandlung der vorerwähnten Hauterkrankungen sind unter anderem Salicylschweineschmalz, Salicyleucerin und Salicylgel in Gebrauch.

Aus der Veröffentlichung von U. Adams und F. Neuwald in Pharm. Ind. Bd. 44, Nr. 6, Seiten 625 bis 629, 1982, ist die Verwendung mittelkettiger Triglycerid-Gele zur Herstellung salicylsäurehaltiger Salben, gegebenenfalls unter Zugabe von Emulgatoren wie Wollwachsalkoholen bekannt. Solche Gele enthalten mittelkettige Triglyceride und ein organisch modifiziertes Magnesiumschichtsilikat als Geliermittel.

In der EP-A-0 063 870 sind entzündungshemmende Salben beschrieben, die entzündungshemmende Substanzen wie Indomethacin, Steroide und dergl. in eine: Salbengrundlage enthalten, welche im wesentlichen mittelkettige Triglyceride (Glyceride von $C_6$-$C_{12}$-Carbonsäuren), ein Carboxyvinylpolymer und Wasser im Verhältnis 1-25 : 0,3-3 : 75 - 99, sowie einen Gelbildner wie Isopropanolamin enthalten.

Darüberhinaus ist es auch bekannt, Lösungen von Salicylsäure in fetten Ölen wie Olivenöl, Ricinusöl und anderen zur Behandlung der vorgenannten Hauterkrankungen zu verwenden.

Durch die DE-A-762 726 ist es bekannt, daß die Löslichkeit von Salicylsäure in Fetten und Ölen, auch Salben, durch den Zusatz von höhermolekularen aliphatischen Alkoholen ($C_8$ - $C_{18}$) oder deren Ethern mit mehrwertigen Alkoholen, die noch mindestens eine freie Hydroxylgruppe enthalten, erhöht werden kann.

Die nicht vorveröffentlichte Anmeldung PCT/EP 88/00649 beschreibt ein Mittel der eingangs genannten Art, das 4 bis 10 Gew.-% Salicylsäure, 1 bis 7 Gew.-% eines aliphatischen 1,2-Diols wie Propan-1,2-diol und 83 bis 95 Gew.-% eines fetten Öls, z.B. ein mittelkettiges Triglycerid enthält, anstelle des fetten Öls aber auch Ester ein- oder mehrwertiger Alkohole mit insbesondere mittelkettigen Fettsäuren enthalten kann.

Im Gebrauch der bekannten salicylsäurehaltigen Mittel hat sich herausgestellt, daß diese bekannten Mittel bei ihrer Anwendung für die so behandelten Patienten aus einer Reihe von Gründen, zu denen beispielsweise eine erhebliche Geruchsbelästigung durch Ranzigwerden der fetten Öle während der Behandlung gehören, nicht oder nur wenig akzeptabel sind und in der Praxis den gewünschten Erfolg eigentlich nur bei stationärer Behandlung bringen. Das ist aber für die mit den Mitteln behandelten Patienten von besonderer Bedeutung, auch aus psychologischen Gründen.

Die Aufgabe der Erfindung besteht darin, ein salicylsäurehaltiges Mittel der eingangs genannten Art zu schaffen, das bei guter Verträglichkeit ohne nachteilhafte Wirkungen für den Patienten ist und eine nichtstationäre Behandlung ermöglicht.

Erfindungsgemäß wird diese Aufgabe dadurch gelöst, daß in dem Mittel 1 Gew.-% bis 15 Gew.-% Salicylsäure, 1 Gew.-% bis 15 Gew.-% eines aliphatischen 1,2-Diols und 70 Gew.-% bis 98 Gew.-% eines Esters eines zweiwertigen Alkohols und mindestens einer Fettsäure, ausgewählt aus der Gruppe der gesättigten mittelkettigen Fettsäuren und der gesättigten langkettigen, verzweigten Fettsäuren, jeweils bezogen auf das Gesamtgewicht des Mittels = 100, enthalten sind.

Weiterhin wird diese Aufgabe nach der Erfindung gelöst durch 1 Gew.-% bis 15 Gew.-% Salicylsäure, 1 Gew.-% bis 15 Gew.-% eines aliphatischen 1,2-Diols und 70 Gew.-% bis 98 Gew.-% eines Triglycerids aus mindestens einer Fettsäure, ausgewählt aus der Gruppe der gesättigten langkettigen, verzweigten Fettsäuren oder deren Mischung mit gesättigten mittelkettigen Fettsäuren, jeweils bezogen auf das Gesamtgewicht des Mittels = 100.

Eine weitere Lösung der Aufgabe ist gekennzeichnet durch Salicylsäure im Bereich von 1 Gew.-% bis 15 Gew.-%, mit Ausnahme des Bereichs von 4 Gew.-% bis 10 Gew.-%, ein aliphatisches 1,2-Diol im Bereich von 1 Gew.-% bis 15 Gew.-%, mit Ausnahme des Bereichs von 1 Gew.-% bis 7 Gew.-%, und ein Triglycerid aus mindestens einer Fettsäure, ausgewählt aus der Gruppe der gesättigten mittelkettigen Fettsäuren, im Bereich von 70 Gew.-% bis 98 Gew.-%, mit Ausnahme des Bereichs von 83 Gew.-% bis 95 Gew.-%, jeweils bezogen auf das Gesamtgewicht des Mittels = 100.

Schließlich besteht die Lösung der Aufgabe auch in der Verwendung einer Zusammensetzung enthaltend 1 Gew.-% bis 15 Gew.-% Salicylsäure, 1 Gew.-% bis 15 Gew.-% eines aliphatischen 1,2-Diols und 70 Gew.-% bis 98 Gew.-% eines Esters eines einwertigen Alkohols und mindestens einer Fettsäure, ausgewählt aus der Gruppe der gesättigten mittelkettigen Fettsäuren und der gesättigten langkettigen, verzweigten Fettsäuren, jeweils bezogen auf das Gesamtgewicht des Mittels = 100, zur Herstellung eines Arzneimittels zur lokalen Therapie von schuppenden Hauterkrankungen im Bereich der Human- und Veterinärmedizin.

Die Sättigungskonzentration der Salicylsäure in fetten Ölen oder deren Mischungen liegt im Bereich von 3 % bis ca. 6 %. Zwar ist Salicylsäure in Ricinusöl bis zu ca. 10 % löslich, jedoch ist dies auf Grund seiner hohen Viskosität und Oxidationsempfindlichkeit (Ranzigwerden) im Rahmen der Erfindung kein brauchbares Lösungsmittel.

Die Löslichkeit von Salicylsäure in aliphatischen 1,2-Diolen ist sehr viel größer und beträgt beispielsweise in Propan-1,2-diol ca. 20 %. Jedoch sind solche aliphatischen 1,2-Diole mit den vorgenannten fetten Ölen nicht mischbar.

Überraschenderweise wurde nun gefunden, daß klare und einphasige Lösungen erhalten werden, wenn man die vorgenannten Ester, aliphatische 1,2-Diole und Salicylsäure in den vorgenannten Mischungsverhältnissen miteinander mischt. Dabei können verschiedene aliphatische 1,2-Diole zur Anwendung kommen, wobei sich Propan-1,2-diol und die verschiedenen Butylenglykole mit benachbarten Hydroxylgruppen als besonders günstig erwiesen haben.

In dem erfindungsgemäßen Mittel können Ester einwertiger oder zweiwertiger Alkohole, insbesondere solche des Propan-1,2-diols, mit gesättigten Fettsäuren mittlerer und größerer Kettenlänge, insbesondere mit geradzahligen und ungeradzahligen, verzweigten oder unverzweigten mittelkettigen ($C_8$ bis $C_{12}$) Fettsäuren oder verzweigten langkettigen ($C_{13}$ bis $C_{18}$) Fettsäuren oder deren Mischungen, oder Triglyceride der gleichen Fettsäuren verwendet werden. Besonders bevorzugt werden dabei einfache oder gemischte Ester von Caprylsäure, Caprinsäure, Nonansäure und Isostearinsäure (Gemisch verzweigtkettiger Octadecansäuren; Fiedler, Lexikon der Hilfsstoffe für Pharmazie, Kosmetik und angrenzende Gebiete, Editio Cantor, Aulendorf, 1981, S. 505). Anstelle der vorgenannten Triglyceride können aber auch gängige fette Öle, bevorzugt pflanzliche fette Öle, eingesetzt werden.

Die erfindungsgemäßen Mittel sind auch bei Langzeitanwendung gut verträglich; bisher wurden auch bei Langzeitanwendung keine allergischen Reaktionen beobachtet. Dabei wird eine hohe Wirksamkeit erreicht; insbesondere wird schon nach relativ kurzer Anwendungszeit, je nach Schwere der Erkrankung, eine völlige Abschuppung, d.h. Erscheinungsfreiheit erreicht, die bei entsprechender Nachbehandlung erhalten bleibt.

Ein besonders wirkungsvolles, bevorzugtes Mittel nach der Erfindung enthält 4 bis 7,5 Gew.-% Salicylsäure, 1 bis 4,5 Gew.-% Propan-1,2-diol und 88 bis 95 Gew.-% Propan-1,2-diolester von Caprylsäure, Caprinsäure oder deren Mischung. Der Anteil an Propan-1,2-diol ist darin so hoch, daß die Salicylsäure fast in gesättigter Lösung vorliegt.

Eine wichtige Ursache für die hohe Wirksamkeit der erfindungsgemäßen Mittel liegt möglicherweise darin, daß die gewählten Lösungsmittelgemische in therapeutisch besonders günstiger Weise auf die erkrankte Haut einwirken und dadurch einen sehr wirksamen Träger bilden, der die Salicylsäure an ihren Wirkungsort bringt.

Weiterhin ist die Behandlung mit den erfindungsgemäßen Mitteln nicht mit unerwünschten und inakzeptablen Belastungen für die Patienten verbunden, weil die erfindungsgemäßen Mittel rasch und vollständig in der Haut resorbiert werden. Es kommt daher nicht zu für den Patienten unerträglichen Geruchsbelästigungen durch Ranzigwerden der Fette oder fetten Öle und nicht zu den unerwünschten Verschmutzungen von Kleidung oder Bettwäsche. Ein weiterer ganz wesentlicher Vorteil der erfindungsgemäßen Mittel besteht darin, daß in den meisten Fällen ein Krankenhausaufenthalt nicht mehr erforderlich ist. Dadurch wurde unsgesamt eine sehr hohe Akzeptanz durch die Patienten erreicht.

Bevorzugte Ausführungsbeispiele der Erfindung werden nachstehend im Zusammenhang mit ihrer Herstellungsvorschrift erläutert.

Salicylsäure wird in Mengen zwischen 1 und 15 Gewichtsteilen mit 1 bis 15 Gewichtsteilen eines aliphatischen 1,2-Diols wie Propan-1,2-diol in üblicher Weise verrieben. Anschließend werden 98 bis 70 Gewichtsteile des jeweils ausgewählten Esters des ein- oder mehrwertigen Alkohols zugegeben; die Mischung wird so lange gerührt, bis eine klare, ölige Lösung entsteht. Dabei wird vorzugsweise eine mit der Salicylsäuremenge entsprechend dem als Lipidphase gewählten Ester zunehmende Menge des aliphatischen 1,2-Diols eingesetzt.

Nach einem bevorzugten Ausführungsbeispiel wird Salicylsäure in Mengen von 4, 5, 7,5 und 10 g abgewogen und jeweils mit einer Menge von 1 bzw. 2 bzw. 4,5 bzw. 7 g Propan-1,2-diol in üblicher Weise verrieben. Anschließend werden zu den 5, 7, 12 und 17 g der Propan-1,2-diol-Verreibung jeweils 95 bzw. 93 bzw. 88 bzw. 83 g eines einfachen oder gemischten Esters von Propan-1,2-diol mit gesättigten mittelkettigen ($C_8$ bis $C_{12}$) Fettsäuren wie Caprylsäure, Caprinsäure oder Nonansäure oder verzweigten langkettigen ($C_{13}$ bis $C_{18}$) Fettsäuren wie Isostearinsäure oder dergleichen gegeben und die Mischung so lange gerührt, bis eine klare, ölige Lösung entsteht. Diese Mittel enthalten somit, jeweils in Gew.-% und bezogen auf das Gesamtgewicht = 100,

| Salicylsäure | 4 | 5 | 7,5 | 10 |
|---|---|---|---|---|
| Propan-1,2-diol | 1 | 2 | 4,5 | 7 |
| Ester | 95 | 93 | 88 | 83 |

Anstelle dieser Ester können auch geeignete Fettalkoholester, fette Öle, besonders pflanzliche fette Öle, eingesetzt werden.

Bevorzugt wird dabei so verfahren, daß - ausgehend von der Sättigungskonzentration der Salicylsäure in dem Ester - gerade so viel Propan-1,2-diol eingesetzt wird, daß eine gesättigte oder nahezu gesättigte Lösung der gewünschten Salicylsäuremenge in dem Propan-1,2-diol-Estergemisch erhalten wird. Jedenfalls hat es sich für die Behandlung als günstig erwiesen, wenn dafür gesättigte oder nahezu gesättigte Lösungen der Salicylsäure verwendet werden.

Diese Mittel wurden in der nachstehend beschriebenen Weise zur Behandlung von schuppenden Hauterkrankungen wie Psoriasis, Tinea Amiantacea, Seborrhoea sicca beim Menschen und von Sommer-Räude und sonstigen schuppenden Erkrankungen beispielsweise bei Ponys eingesetzt. Insgesamt hat sich bei diesen und anderen vergleichbaren Untersuchungen herausgestellt, daß unter dem Gesichtspunkt der Verträglichkeit, der Wirksamkeit und der Behandlungsdauer bis zur Erscheinungsfreiheit die Mittel, die 4 Gew.-% bis 7,5 Gew.-% Salicylsäure, 1 Gew.-% bis 4,5 Gew.-% des aliphatischen 1,2-Diols und 88 Gew.-% bis 93 Gew.-% des Esters enthielten, die relativ beste Wirkung zeigten.

Bei weiteren Untersuchungen wurde gefunden, daß anstelle von Propan-1,2-diol auch die verschiedenen Butylenglykole mit benachbarten Hydroxylgruppen und gegebenenfalls auch andere aliphatische 1,2-Diole eingesetzt werden können.

Die therapeutische Anwendung eines Ausführungsbeispiels, das 5 Gew.-% Salicylsäure, 2 Gew.-% Propan-1,2-diol und 93 Gew.-% mittelkettige Triglyceride (Gesamtgewicht des Mittels = 100) enthält, wird nachfolgend an Hand einer Patientengruppe von 150 Personen erläutert, die hauptsächlich an Psoriasis und zu einem kleineren Teil an anderen schuppenden Haut- und Kopfhauterkrankungen, z.B. seborrhoeischen Ekzemen litten. Die Behandlung bestand darin, daß

a) bei Erkrankungen der Kopfhaut

das Mittel zwei- bis dreimal wöchentlich auf die befallenen Stellen der Kopfhaut aufgetragen wird; die Behandlung kann unter Benutzung einer Dusch-Plastikhaube mit schließendem Gummirand und einer handelsüblichen Operationshaube zur Fixierung über Nacht erfolgen, und

b) bei Erkrankungen am Körper

das Mittel ein- bis zweimal täglich auf die befallenen Stellen aufgetragen wird; nach 10 bis 15 Minuten Einwirkungszeit ist das Mittel von der Haut aufgenommen.

Bei 8 Personen aus der Patientengruppe wurde die Behandlung vorzeitig abgebrochen aus Gründen, die nichts mit der eigentlichen Behandlung zu tun hatten. Bei 50 Personen aus der Patientengruppe wurde in Abhängigkeit von der Schwere der ursprünglichen Erkrankung totale Abschuppung und damit die erwünschte Erscheinungsfreiheit nach einer Behandlungszeit von

1 Monat bei leichter ursprünglicher Erkrankung,

bis zu 3 Monaten bei mittlerer ursprünglicher Erkrankung und

bis zu 4 Monaten bei schwerer ursprünglicher Erkrankung

erzielt, wobei der Beginn der Abschuppung bereits in den ersten Wochen der Behandlung einsetzte.

Bei den meisten der restlichen 92 Patienten war die Abschuppung nach bis zu einjähriger Behandlungsdauer sehr weit fortgeschritten und läßt auch hier totale Abschuppung und damit die erwünschte Erscheinungsfreiheit erwarten. Allergische Reaktionen wurden nicht beobachtet, und die Behandlung wurde von den Patienten hervorragend akzeptiert. Dazu trägt sicher bei, daß das Mittel geruchsneutral ist und schnell in die Haut einzieht, wodurch keine Verschmutzung von Kleidung oder Bettwäsche erfolgt, sowie die Tatsache, daß nur bei besonderen Umständen und sehr schweren Erkrankungen ein Klinikaufenthalt erforderlich wird.

Gleiche oder ähnliche Wirkungen werden erzielt, wenn die Triglyceride durch andere der vorgenannten Ester ersetzt werden.

Als sehr vorteilhafte Nebenwirkung wurde deutlich erkennbarer vermehrter Haarwuchs, auch im veterinär-medizinischen Bereich, festgestellt.

Die einmal erreichte Erscheinungsfreiheit kann auch in der Folgezeit aufrechterhalten werden. Dazu empfiehlt sich eine Folgetherapie unter Verwendung handelsüblicher, eine erneute Schuppenbildung reduzierender Mittel wie Dithranol im Wechsel mit den zuvor beschriebenen Mitteln.

**Patentansprüche**

1.  Salicylsäurehaltiges Mittel zur lokalen Therapie von schuppenden Hauterkrankungen im Bereich der Human- und Veterinärmedizin,
gekennzeichnet durch
1 Gew.-% bis 15 Gew.% Salicylsäure
1 Gew.-% bis 15 Gew.-% eines aliphatischen 1,2-Diols und
70 Gew.% bis 98.-% eines Esters eines zweiwertigen Alkohols und mindestens einer Fettsäure, ausgewählt aus der Gruppe der gesättigten mittelkettigen Fettsäuren und der gesättigten langkettigen, verzweigten Fettsäuren, jeweils bezogen auf das Gesamtgewicht des Mittels = 100.

2.  Mittel nach Anspruch 1, dadurch gekennzeichnet, daß darin eine mit der Konzentration der Salicylsäure zunehmende Konzentration des aliphatischen 1,2-Diols enthalten ist.

3.  Mittel nach Anspruch 1 oder 2, dadurch gekennzeichnet, daß die aliphatischen 1,2-Diole aus der Gruppe Propan-1,2-diol und Butylenglykol mit benachbarten Hydroxylgruppen ausgewählt sind.

4.  Mittel nach einem der Ansprüche 1 bis 3, dadurch gekennzeichnet, daß der Ester aus Propan-1,2-diol gebildet ist.

5.  Mittel nach Anspruch 4, dadurch gekennzeichnet, daß das Mittel 4 Gew.-% bis 7,5 Gew.-% Salicylsäure, 1 Gew.-% bis 4,5 Gew.-% Propan-1,2-diol und 88 Gew.-% bis 95 Gew.-% eines mittelkettigen gesättigten Propan-1,2-diolfettsäureesters enthält, jeweils bezogen auf das Gesamtgewicht des Mittels = 100.

6.  Mittel nach einem der Ansprüche 1 bis 5, dadurch gekennzeichnet, daß die gesättigten mittelkettigen Fettsäuren aus Caprylsäure, Caprinsäure, Nonansäure oder deren Mischung ausgewählt sind.

7.  Mittel nach einem der Ansprüche 1 bis 5, dadurch gekennzeichnet, daß die gesättigte langkettige, verzweigte Fettsäure Isostearinsäure ist.

8.  Mittel nach einem der Vorstehenden Ansprüche, dadurch gekennzeichnet, daß der Anteil an dem aliphatischen 1,2-Diol so gewählt ist, daß die Salicylsäure wenigstens in nahezu gesättigter Lösung vorliegt.

9.  Salicylsäurehaltiges Mittel zur lokalen Therapie von schuppenden Hauterkrankungen im Bereich der Human- und Veterinärmedizin,
gekennzeichnet durch
1 Gew.-% bis 15 Gew.-% Salicylsäure
1 Gew.-% bis 15 Gew.-% eines aliphatischen 1,2-Diols und
70 Gew.-% bis 98 Gew.-% eines Triglycerids aus mindestens einer Fettsäure, ausgewählt aus der Gruppe der gesättigten langkettigen, verzweigten Fettsäuren oder deren Mischung mit gesättigten mittelkettigen Fettsäuren, jeweils bezogen auf das Gesamtgewicht des Mittels = 100.

10. Mittel nach Anspruch 9, dadurch gekennzeichnet, daß darin eine mit der Konzentration der Salicylsäure zunehmende Konzentration des aliphatischen 1,2-Diols enthalten ist.

11. Mittel nach Anspruch 9 oder 10, dadurch gekennzeichnet, daß die aliphatischen 1,2-Diole aus der Gruppe Propan-1,2-diol und Butylenglykol mit benachbarten Hydroxylgruppen ausgewählt sind.

12. Mittel nach einem der Ansprüche 9 bis 11, dadurch gekennzeichnet, daß die gesättigten mittelkettigen Fettsäuren aus Caprylsäure, Caprinsäure, Nonansäure oder deren Mischung ausgewählt sind.

13. Mittel nach einem der Ansprüche 9 bis 12, dadurch gekennzeichnet, daß die gesättigte langkettige, verzweigte Fettsäure Isostearinsäure ist.

14. Mittel nach einem der Ansprüche 9 bis 13, dadurch gekennzeichnet, daß der Anteil an dem aliphatischen 1,2-Diol so gewählt ist, daß die Salicylsäure wenigstens in nahezu gesättigter Lösung vorliegt.

15. Salicylsäurehaltiges Mittel zur lokalen Therapie von schuppenden Hauterkrankungen im Bereich der Human- und Veterinärmedizin,
gekennzeichnet durch

Salicylsäure im Bereich von 1 Gew.-% bis 15 Gew.-%, mit Ausnahme des Bereichs von 4 Gew.-% bis 10 Gew.-%, ein aliphatisches 1,2-Diol im Bereich von 1 Gew.-% bis 15 Gew.-%, mit Ausnahme des Bereichs von 1 Gew.-% bis 7 Gew.-%, und

ein Triglycerid aus mindestens einer Fettsäure, ausgewählt aus der Gruppe der gesättigten mittelkettigen Fettsäuren, im Bereich von 70 Gew.-% bis 98 Gew.-%, mit Ausnahme des Bereichs von 83 Gew.-% bis 95 Gew.-%, jeweils bezogen auf das Gesamtgewicht des Mittels = 100.

16. Mittel nach Anspruch 15, dadurch gekennzeichnet, daß darin eine mit der Konzentration der Salicylsäure zunehmende Konzentration des aliphatischen 1,2-Diols enthalten ist.

17. Mittel nach Anspruch 15 oder 16, dadurch gekennzeichnet, daß die aliphatischen 1,2-Diole aus der Gruppe Propan-1,2-diol und Butylenglykol mit benachbarten Hydroxylgruppen ausgewält sind.

18. Mittel nach einem der Ansprüche 15 bis 17, dadurch gekennzeichnet, daß die gesättigten mittelkettigen Fettsäuren aus Caprylsäure, Caprinsäure, Nonansäure oder deren Mischung ausgewählt sind.

19. Mittel nach einem der Ansprüche 15 bis 18, dadurch gekennzeichnet, daß der Anteil an dem aliphatischen 1,2-Diol so gewählt ist, daß die Salicylsäure wenigstens in nahezu gesättigter Lösung vorliegt.

20. Mittel nach einem der Ansprüche 1 bis 8, gekennzeichnet durch Zusatz eines die erneute Schuppenbildung reduzierenden Mittels.

21. Mittel nach einem der Ansprüche 9 bis 14, gekennzeichnet durch Zusatz eines die erneute Schuppenbildung reduzierenden Mittels.

22. Mittel nach einem der Ansprüche 15 bis 19, gekennzeichnet durch Zusatz eines die erneute Schuppenbildung reduzierenden Mittels.

23. Verwendung einer Zusammensetzung enthaltend
     1 Gew.-% bis 15 Gew.-% Salicylsäure,
     1 Gew.-% bis 15 Gew.-% eines aliphatischen 1,2-Diols und
70 Gew.-% bis 98 Gew.-% eines Esters eines einwertigen Alkohols und mindestens einer Fettsäure, ausgewählt aus der Gruppe der gesättigten mittelkettigen Fettsäuren und der gesättigten langkettigen, verzweigten Fettsäuren, jeweils bezogen auf das Gesamtgewicht des Mittels = 100,
zur Herstellung eines Arzneimittels zur lokalen Therapie von schuppenden Hauterkrankungen im Bereich der Human- und Veterinärmedizin.


## Claims

1. Salicylic acid containing agent for local therapy of lepidosis in the field of human and veterinary medicine, characterised by
1 percent to 15 percent of salicylic acid,
1 percent to 15 percent of an aliphatic 1,2-diol, and
70 percent to 98 percent of an ester formed by a dihydric alcohol and at least one fatty acid selected from the group consisting of saturated medium-chain fatty acids and saturated long-chain, branched fatty acids, each in percent by weight of the agent.

2. Agent according to claim 1, characterised in that the concentration of the aliphatic 1,2-diol contained therein increases with increasing concentration of salicylic acid.

3. Agent according to claim 1 or 2, characterised in that the aliphatic 1,2-diols are selected from the group consisting of propane 1,2-diol and butylene glycol containing vicinal hydroxy groups.

4. Agent according to any one of claims 1 to 3, characterised in that the ester is formed from propane 1,2-diol.

5. Agent according to claim 4, characterised in that the agent contains salicylic acid in the range of 4 percent to 7.5 percent, propane 1,2-diol in the range of 1 percent to 4.5 percent, and propane 1,2-diol ester of a medium-chain saturated fatty acid in the range of 88 percent to 95 percent, each in percent by weight of the agent.

6. Agent according to any one of claims 1 to 5, characterised in that the saturated medium-chain fatty acids are selected from caprylic acid, capric acid, nonanoic acid or mixtures thereof.

7. Agent according to any one of claims 1 to 5, characterised in that the saturated long-chain, branched fatty acid is isostearic acid.

8. Agent according to any one of the preceding claims, characterised in that the proportion of the aliphatic 1,2-diol is selected such that the salicylic acid is present in at least almost saturated solution.

9. Salicylic acid containing agent for local therapy of lepidosis in the field of human and veterinary medicine, characterised by
1 percent to 15 percent of salicylic acid,
1 percent to 15 percent of an aliphatic 1,2-diol, and
70 percent to 98 percent of a triglyceride of at least one fatty acid selected from the group consisting of saturated long-chain, branched fatty acids or their mixture with saturated medium-chain fatty acids, each in percent by weight of the agent.

10. Agent according to claim 9, characterised in that the concentration of the aliphatic 1,2-diol contained therein increases with increasing concentration of salicylic acid.

11. Agent according to claim 9 or 10, characterised in that the aliphatic 1,2-diols are selected from the group consisting of propane 1,2-diol and butylene glycol containing vicinal hydroxy groups.

12. Agent according to any one of claims 9 to 11, characterised in that the saturated medium-chain fatty acids are selected from caprylic acid, capric acid, nonanoic acid or mixtures thereof.

13. Agent according to any one of claims 9 to 12, characterised in that the saturated long-chain, branched fatty acid is isostearic acid.

14. Agent according to any one of claims 9 to 13, characterised in that the proportion of the aliphatic 1,2-diol is selected such that salicylic acid is present in at least almost saturated solution.

15. Salicylic acid containing agent for local therapy of lepidosis in the field of human or veterinary medicine characterised by
salicylic acid in the range of 1 percent to 15 percent with the exception of the range of 4 percent to 10 percent,
an aliphatic 1,2-diol in the range of 1 percent to 15 percent with the exception of the range of 1 percent to 7 percent, and
a triglyceride of at least one fatty acid selected from the group consisting of saturated medium-chain fatty acids in the range of 70 percent to 98 percent with the exception of the range of 83 percent to 95 percent, each in percent by weight of the agent.

16. Agent according to claim 15, characterised in that the concentration of the aliphatic 1,2-diol contained therein increases with increasing concentration of salicylic acid.

17. Agent according to claim 15 or 16, characterised in that the aliphatic 1,2-diols are selected from the group consisting of propane 1,2-diol and butylene glycol containing vicinal hydroxy groups.

18. Agent according to any one of claims 15 to 17, characterised in that the saturated medium-chain fatty acids are selected from caprylic acid, capric acid, nonanoic acid or mixtures thereof.

19. Agent according to any one of claims 15 to 18, characterised in that the proportion of the aliphatic 1,2-diol is selected such that salicylic acid is present in at least almost saturated solution.

20. Agent according to any one of claims 1 to 8, characterised by the addition of an agent reducing new lepidosis formation.

21. Agent according to any one of claims 9 to 14, characterised by the addition of an agent reducing new lepidosis formation.

22. Agent according to any one of claims 15 to 19, characterised by the addition of an agent reducing new lepidosis formation.

23. Method of using a composition containing
1 percent to 15 percent of salicylic acid,
1 percent to 15 percent of an aliphatic 1,2-diol, and
70 percent to 98 percent of an ester formed by a monohydric alcohol and at least one fatty acid selected from the group consisting of saturated medium-chain fatty acids and saturated long-chain, branched fatty acids, each in percent by weight of the agent,
for the manufacture of a medicament for local therapy of lepidosis in the field of human and veterinary medicine.

## Revendications

1. Agent comprenant de l'acide salicylique destiné à la thérapie locale d'affections de la peau écailleuses dans le domaine de la médecine humaine et vétérinaire,
   caractérisé par
   1% en poids à 15% en poids d'acide salicylique
   1% en poids à 15% en poids d'un 1,2-diol aliphatique, et
   70% en poids à 98% en poids d'un ester d'un dialcool et au moins d'un acide gras choisi du groupe des acides gras saturés à chaîne moyenne et des acides gras saturés ramifiés à chaîne longue, respectivement par référence au poides total de l'agent = 100.

2. Agent selon la revendication 1, caractérisé par le fait qu'une concentration de 1,2-diol aliphatique s'accroissant avec la concentration de l'acide salicylique y est comprise.

3. Agent selon la revendication 1 ou 2, caractérisé par le fait que les 1,2-diols aliphatiques sont choisis du groupe 1,2-diol de propane et glycols de butylène ayant des groupes d'hydroxyles contigus.

4. Agent selon l'une des revendications 1 à 3, caractérisé par le fait que l'ester est formé d'un 1,2-diol de propane.

5. Agent selon la revendication 4, caractérisé par le fait que l'agent comprend 4 à 7,5% en poids d'acide salicylique, 1 à 4,5% en poids de 1,2-diol de propane et 88 à 95% en poids d'un ester d'acide gras de 1,2-diol de propane saturé à chaîne moyenne, respectivement par référence au poids total de l'agent = 100.

6. Agent selon l'une des revendications 1 à 5, caractérisé par le fait que les acides gras saturés à chaîne moyenne sont choisis d'acide caprylique, d'acide caprique, d'acide de nonane ou d'une mélange de ceux-ci.

7. Agent selon l'une des revendications 1 à 5, caractérisé par le fait que l'acide gras saturé ramifié à chaîne longue est l'acide d'isostéarine.

8. Agent selon l'une des revendications ci-dessus, caractérisé par le fait que la part du 1,2-diol aliphatique est choisie de sorte que l'acide salicylique est présent en solution au moins approximativement saturée.

9. Agent comprenant de l'acide salicylique destiné à la thérapie locale d'affections de la peau écailleuses dans le domaine de la médecine humaine et vétérinaire,
   caractérisé par
   1% en poids à 15% en poids d'acide salicylique
   1% en poids à 15% en poids d'un 1,2-diol aliphatique, et
   70% en poids à 98% en poids d'un triglycéride au moins d'un acide gras choisi du groupe des acides gras saturés ramifiés à chaîne longue ou d'une mélange de ceux-ci avec des des acides gras saturés à chaîne moyenne et, respectivement par référence au poides total de l'agent = 100.

10. Agent selon la revendication 9, caractérisé par le fait qu'une concentration du 1,2-diol aliphatique s'acroissant avec la concentration de l'acide salicylique y est comprise.

11. Agent selon la revendication 9 ou 10, caractérisé par le fait que les 1,2-diols aliphatiques sont choisis du groupe 1,2-diol de propane et glycols de butylène ayant des groupes d'hydroxyles contigus.

12. Agent selon l'une des revendications 9 à 11, caractérisé par le fait que les acides gras saturés à chaîne moyenne sont choisis d'acide caprylique, d'acide caprique, d'acide de nonane ou d'une mélange de ceux-ci.

13. Agent selon l'une des revendications 9 à 12, caractérisé par le fait que l'acide gras saturé ramifié à chaîne longue est l'acide d'isostéarine.

14. Agent selon l'une des revendications 9 à 13, caractérisé par le fait que la part du 1,2-diol aliphatique est choisie de sorte que l'acide salicylique est présent en solution au moins approximativement saturée.

15. Agent comprenant de l'acide salicylique destiné à la thérapie locale d'affections de la peau écailleuses dans le domaine de la médecine humaine et vétérinaire,
   caractérisé par
   de l'acide saliclique dans le domaine de 1% en poids à 15% en poids à l'exception du domaine de 4% en poids à 10% en poids,
   un 1,2-diol aliphatique dans le domaine de 1% en poids à 15% en poids à l'exception du domaine de 1% en poids à 7 % en poids, et
   un triglycéride au moins d'un acide gras choisi du groupe des des acides gras saturés à chaîne moyenne dans le domaine de 70% en poids à 98% en poids à l'exception du domaine de 83% en poids à 95% en poids, respectivement par référence au poides total de l'agent = 100.

16. Agent selon la revendication 15, caractérisé par le fait qu'une concentration du 1,2-diol aliphatique s'accroissant avec la concentration de l'acide salicylique y est comprise.

17. Agent selon la revendication 15 ou 16, caractérisé par le fait que les 1,2-diols aliphatiques sont choisis du groupe 1,2-diol de propane et glycols de butylène ayant des groupes d'hydroxyles contigus.

18. Agent selon l'une des revendications 15 à 17, caractérisé par le fait que les acides gras saturés à chaîne moyenne sont choisis d'acide caprylique, d'acide caprique, d'acide de nonane ou d'une mélange de ceux-ci.

19. Agent selon l'une des revendications 15 à 18, caractérisé par le fait que la part du 1,2-diol aliphatique est choisie de sorte que l'acide salicylique est présent en solution au moins approximativement saturée.

20. Agent selon l'une des revendications 1 à 8, caractérisé par l'addition d'un agent réduisant la nouvelle formation d'écailles.

21. Agent selon l'une des revendications 9 à 14, caractérisé par l'addition d'un agent réduisant la nouvelle formation d'écailles.

22. Agent selon l'une des revendications 15 à 19, caractérisé par l'addition d'un agent réduisant la nouvelle formation d'écailles.

23. Emploi d'une composition, comprenant
   1% en poids à 15% en poids d'acide salicylique
   1% en poids à 15% en poids d'un 1,2-diol aliphatique. et
   70% en poids à 98% en poids d'un ester d'un monoalcool et au moins d'un acide gras choisi du groupe des acides gras saturés à chaîne moyenne et des acides gras saturés ramifiés à chaîne longue, respectivement par référence au poides total de l'agent = 100,
   afin de produire un médicament destiné à la thérapie locale d'affections de la peau écailleuses dans le domaine de la médecine humaine et vétérinaire.

9